# EUROPÄISCHE PATENTSCHRIFT

(11) **EP 3 958 799 B1**
(45) Veröffentlichungstag und Bekanntmachung des Hinweises auf die Patenterteilung: **13.09.2023**
(21) Anmeldenummer: 20721583.1
(22) Anmeldetag: 24.04.2020
(51) Int. Cl.: A61F 5/37, A47G 9/02, A61G 7/00, A61G 7/05

(54) **PATIENTEN-IMMOBILISATIONSVORRICHTUNG, SYSTEM UND VERFAHREN ZUR RUHIGSTELLUNG EINES PATIENTEN**
PATIENT IMMOBILIZATION APPARATUS, SYSTEM AND METHOD FOR IMMOBILIZING A PATIENT
DISPOSITIF D'IMMOBILISATION D'UN PATIENT, SYSTÈME ET PROCÉDÉ DE MISE AU REPOS D'UN PATIENT

(30) Priorität: 26.04.2019 US 201962839420 P
(43) Veröffentlichungstag der Anmeldung: 02.03.2022
(73) Patentinhaber: Isys Medizintechnik GmbH, 6370 Kitzbühel (AT)
(72) Erfinder: VOGELE, Michael, 6370 Kitzbühel (DE)
(74) Vertreter: Keller Schneider Patentanwaltsgesellschaft mbH
(86) Internationale Anmeldenummer: PCT/EP2020/061563
(87) Internationale Veröffentlichungsnummer: WO 2020/216951

(56) Entgegenhaltungen:
- US-A- 4 180 879
- US-A- 4 908 889
- US-A- 5 626 397

## Beschreibung

Die Erfindung betrifft eine Patienten-Immobilisationsvorrichtung, sowie ein System und ein Verfahren zur Ruhigstellung eines Patienten, insbesondere während der Durchführung eines medizinischen Verfahrens, wie in den Ansprüchen definiert.

### STAND DER TECHNIK

Bei vielen medizinischen Verfahren ist es erforderlich, dass ein Patient einen Körperteil, der dem medizinischen Verfahren unterzogen wird, nicht bewegt. Zu den medizinischen Verfahren, die eine Ruhigstellung des Patienten erfordern, gehören beispielsweise die diagnostische und therapeutische Radiologie, die Strahlentherapie, operative/chirurgische Verfahren sowie die prä- oder postoperative Versorgung.

In der veröffentlichten Patentanmeldung US 2017/0246024 A1 des Anmelders wird eine einfache Vorrichtung zur Ruhigstellung des menschlichen Körpers oder von Körperteilen offenbart. Die Vorrichtung verfügt über mindestens ein Immobilisierungselement, das auf der Körperoberfläche positioniert werden kann. Das Immobilisierungselement besteht aus einem verfestigten, nicht-gewebten Vliesstoff, der mit einem Mikro-Klettverschluss befestigt werden kann. US5626397 offenbart eine Patienten-Immobilisationsvorrichtung umfassend ein flächiges Element als länglichen Mittelteil und eine Vielzahl von separaten Armen, die sich seitlich auf beiden Seiten des Mittelteils erstrecken.

Aufgabe der Erfindung ist es, eine verbesserte Patienten-Immobilisationsvorrichtung in Verbindung mit einem System und einem Verfahren bereitzustellen, dass eine noch einfachere und flexiblere Handhabung erlaubt.

### OFFENBARUNG DER ERFINDUNG

Eine verbesserte Patienten-Immobilisationsvorrichtung umfasst ein flächiges Element aus Vliesstoff. Das flächige Element hat einen länglichen Mittelteil und eine Vielzahl von Armen, die sich seitlich auf beiden Seiten vom Mittelteil aus erstrecken. Durch eine Mehrzahl von Armen ist eine besonders flexible und bei Bedarf auch großflächige Ruhigstellung von Patienten und deren Körperteilen möglich. Als Mehrzahl oder Vielzahl von Armen ist dabei eine Anzahl von wenigstens zwei Armen auf beiden Seiten des Mittelteils zu verstehen.

Die Patienten-Immobilisationsvorrichtung kann dem Endbenutzer besonders vorteilhaft in gefaltetem Zustand zur Verfügung gestellt werden. Im gefalteten Zustand ist jeder der Arme seitlich auf den länglichen Mittelteil gefaltet. Teile des länglichen Mittelteils mit den seitlich daran gefalteten Armen sind weiter bevorzugt in Längsrichtung aufeinander gefaltet. Zusätzlich kann jeder Arm bevorzugt auch noch seitlich auf sich selbst gefaltet werden, bevor er seitlich auf den länglichen Mittelteil gefaltet wird. Insgesamt entsteht durch die vielfältigen Faltmöglichkeiten ein kompaktes, auch bei großflächigeren Anwendungen leicht handhabbares Paket

Ein System zur Immobilisierung eines Patienten umfasst die Patienten-Immobilisierungsvorrichtung und eine Vielzahl von Mikro-Hakenbefestigungselementen, die an den Seiten eines Patiententisches befestigbar sind. Ein oder mehrere Arme der Patienten-Immobilisierungsvorrichtung können durch Mikro-Haken gehalten werden, die an Teilen der Mikro-Haken-Befestigungselemente angeordnet sind.

Die Mikro-Hakenbefestigungselemente umfassen bevorzugt ein plattenförmiges Teil mit einer Vielzahl von darauf angeordneten Mikro-Haken. Das Mikro-Haken Befestigungselement umfasst ferner einen oberen und einen unteren Steg. Der obere Steg und der untere Steg sind so konfiguriert und beabstandet, dass eine Schiene eines Patiententisches zwischen diesen eingeklemmt werden kann. Der untere Steg kann einen Kanal enthalten, der so konfiguriert ist, dass er einen unteren Teil der Schiene des Patiententisches aufnimmt, sowie einen verformbaren Gummistreifen, der in dem Kanal angeordnet ist.

Eine vorteilhafte Weiterentwicklung des erfindungsgemäßen Systems sieht vor, dass Einweg-Pflaster zwischen der Patienten-Immobilisationsvorrichtung und den Mikro-Hakenbefestigungselementen angeordnet werden können. Die Einweg-Pflaster haben auf der Innenseite Stoffschlaufen und auf der Außenseite Mikrohaken angeordnet. Die Einweg-Pflaster können in einer Sandwich-Struktur zwischen den Mikro-Haken-Befestigungselementen und dem flächigen Element aus Vliesstoff der Patienten-Immobilisationsvorrichtung verwendet werden.

Ein alternatives Mikro-Haken-Befestigungselement umfasst eine Kederschnur und einen um die Kederschnur gewickeltes Mikro-Hakenpflaster. Die Kederschnur kann einen unteren Teil mit einem Querschnittsdurchmesser aufweisen, der grösser als der Durchmesser eines Kederschienenschlitzes eines Patiententisches ist, und einen oberen Teil mit einer Breite, die kleiner als der Durchmesser des Kederschienenschlitzes des Patiententisches ist. Der obere Teil der Kederschnur kann optional in seinem durch den Kederschienenschlitz herausgeführten Bereich zwischen 45 und 90 Grad gebogen werden.

Jedes der Mikro-Haken-Befestigungselemente kann mit einem abnehmbaren Einweg-Pflaster versehen sein, das in die Mikro-Haken der Mikro-Haken-Befestigungselemente eingreift. Das abnehmbare Einweg-Pflaster kann vorgesehen werden, um die Mikrohaken vor dem Gebrauch zu schützen oder um einen direkten Kontakt zwischen den Mikrohaken und dem Patienten zu verhindern.

Ein Verfahren zur Sicherung eines Patienten auf einem Patiententisch kann auf der Bereitstellung der Patienten-Immobilisierungsvorrichtung basieren. Die Patienten-Immobilisationsvorrichtung wird auf den Patienten gelegt und zwei oder mehr Arme der Patienten-Immobilisationsvorrichtung werden an Mikrohaken-Befestigungselementen an gegenüberliegenden Seiten des Patiententisches befestigt. Das Verfahren kann ferner die selektive Entfernung eines Teils der Patienten-Immobilisierungsvorrichtung umfassen, um eine Zugangsöffnung zur Durchführung eines medizinischen Eingriffs am Patienten zu schaffen. Die Patienten-Immobilisierungsvorrichtung weist zu diesem Zweck bevorzugt Perforationen auf, an denen einzelne Arme und/oder Teile des Mittelteils ganz oder teilweise abtrennbar sind.

Die folgende detaillierte Beschreibung der Erfindung hat lediglich beispielhaften Charakter und ist nicht dazu gedacht, die Erfindung oder die Anwendung und Verwendungen der Erfindung einzuschränken. Darüber hinaus besteht nicht die Absicht, sich an eine Theorie zu binden, die im vorhergehenden Hintergrund der Erfindung oder in der folgenden detaillierten Beschreibung der Erfindung dargestellt wird.

### KURZE BESCHREIBUNG DER ZEICHNUNGEN

- Fig. 1: zeigt eine flache Ansicht einer Patienten-Immobilisierungsvorrichtung,
- Fig. 2: zeigt das Zusammenklappen einer Patienten-Immobilisierungsvorrichtung gemäß Figur 1,
- Fig. 3: zeigt die Patienten-Immobilisationsvorrichtung gemäß Figur 1 und 2 im zusammengeklappten Zustand,
- Fig. 4: zeigt die Patienten-Immobilisierungsvorrichtung von Figur 1 im Gebrauch an einem auf einen Patiententisch liegenden Patienten,
- Fig. 5: zeigt eine perspektivische Vorder- und Seitenansicht eines Mikro-Hakenbefestigungselements für ein System zur Patienten-Immobilisierung, das an einer Schiene eines Patiententisches befestigt ist,
- Fig. 6: zeigt eine perspektivische Rück- und Seitenansicht eines Mikro-Hakenbefestigungselements gemäß Figur 5,
- Fig. 7: zeigt eine perspektivische Rück- und Seitenansicht eines alternativen Befestigungselements,
- Fig. 8: zeigt eine perspektivische Vorder- und Seitenansicht, die das in eine Kederschiene eines Patiententisches eingesetzte alternative Befestigungselement gemäß Fig. 7 in Gebrauch zeigt,
- Fig. 9: zeigt eine Rück- und Seitenansicht eines alternativen Befestigungselements gemäß den Figuren 7 und 8.
- Fig. 10: zeigt Querschnitte alternativer Konfigurationen von Kederschnüren, die in den alternativen Befestigungselementen gemäß Fig. 7 bis 9 verwendbar sind,
- Fig. 11: zeigt Querschnitte alternativer Ausführungsformen von Befestigungselementen innerhalb von Kederschienen, und
- Fig. 12: zeigt eine Explosionsschnittdarstellung eines Befestigungselements mit einem Ankerpflaster, einem Einweg-Pflaster und der Patienten-Immobilisierungsvorrichtung.

### AUSFÜHRUNGSFORMEN DER ERFINDUNG

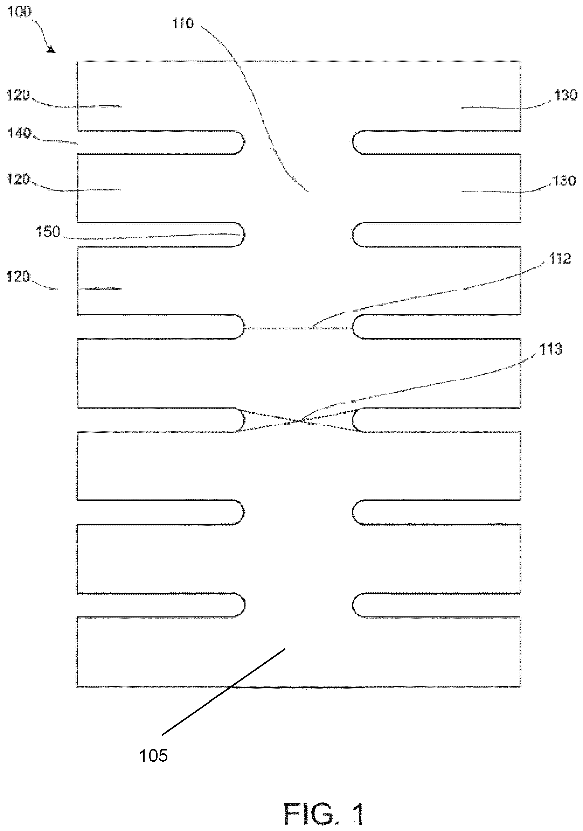

Unter Bezugnahme auf Figur 1 wird eine nicht-gewebte, ein flächiges Element 105 bildende Vliesstofflage zu einer Patienten-Immobilisationsvorrichtung 100 geformt. Die Immobilisierungsvorrichtung 100 hat einen länglichen Mittelteil 110, von dem sich zwei oder mehr Arme 120, 130 seitlich auf beiden Seiten des Mittelteils 110 erstrecken. Das Gewebe des flächigen Elements 105 bzw. der nicht-gewebten Vliesstofflage kann bevorzugt aus nicht gewebtem Polypropylen mit einer Dicke von etwa 1 mm, vorzugsweise von etwa 0,8 mm, hergestellt werden. Um die gewünschte Festigkeit zu erreichen, sollte das Gewebe vorzugsweise eine Zugfestigkeit von mehr als 100 N je 50 mm Gewebebreite aufweisen. Das Gewebe ist röntgenstrahlendurchlässig und MR (Magnetresonanztomographie) -sicher. Es kann mit Dampf oder Ethylenoxid gemäß der Standards ISO 11135, ISO 10993-7 oder EN 1422 sterilisiert werden.

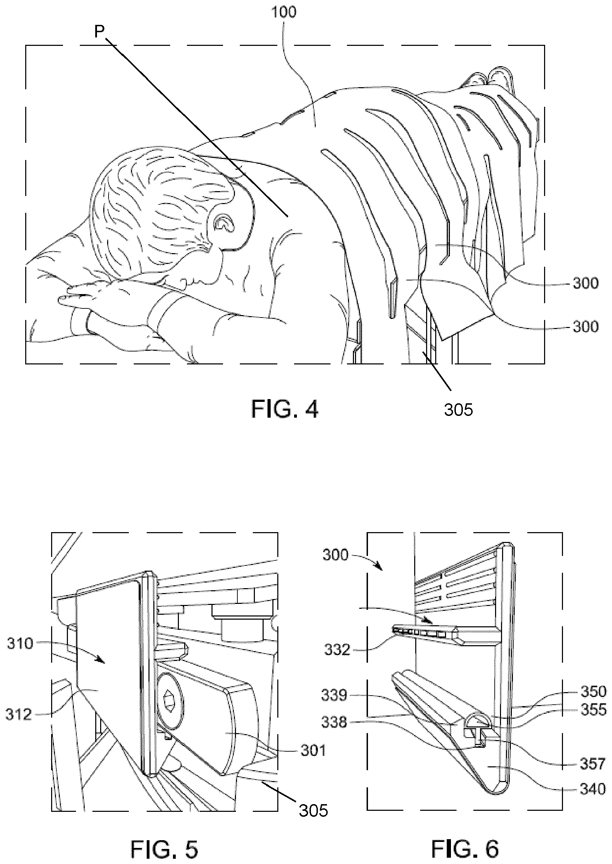

Die Patienten-Immobilisationsvorrichtung kann, wie in Figur 4 dargestellt, zur Fixierung eines Patienten P an einem Patiententisch 305 verwendet werden, indem der Mittelteil 110 auf den Rumpf des Patienten gelegt und die Arme 120, 130 an Mikro-Hakenbefestigungselementen 300 befestigt werden, die an jeder Seite des Patiententisches 305 befestigbar sind. Andere Verwendungen der Patienten-Immobilisationsvorrichtung 100 sind möglich. Dazu gehört die Immobilisierung eines Körperteils eines Patienten P mit der Patienten-Immobilisationsvorrichtung 100 ähnlich einer Bandage oder eines Gipsverbandes, möglicherweise zusammen mit einem oder mehreren starren, länglichen Elementen, beispielsweise beim Schienen eines Knochenbruchs

Der längliche Mittelteil 110 ist vorzugsweise zwischen 100 cm und 200 cm lang und hat eine Breite zwischen 10 cm und 30 cm. Der längliche Mittelteil 110 kann geeignet sein, einen wesentlichen Teil des Rumpfes eines Patienten P abzudecken, so dass eine gleichmäßige Druckausübung auf den Rumpf möglich ist, ohne lokale Druckpunkte zu erzeugen.

Die Arme 120 bzw. 130 sind vorzugsweise zwischen 5 cm und 20 cm breit und zwischen 50 cm und 150 cm lang. Der längliche Mittelteil 110 ist in der Regel länger als die Arme 120, 130 und hat häufig ungefähr die gleiche Breite wie die Arme 120, 130. Zwischen je zwei benachbarten Armen 120, 130 können Längslücken 140 ausgeschnitten werden. Die Längslücken 140 können zwischen 1 cm und 10 cm breit sein. Die Längslücken 140 können mit halbkreisförmigen Übergängen 150 ausgeschnitten werden, die die Arme 120, 130 am Mittelteil 110 trennen. Dadurch wird die Gefahr eines unbeabsichtigten Reißens der Stoffbahn verringert.

Die Arme 120, 130 können symmetrisch angeordnet werden, wobei jeweils ein linker Arm 120 gegenüber einem rechten Arm 130 symmetrisch am Mittelteil 110 angeordnet ist. Die Patienten-Immobilisationsvorrichtung 100 kann zwischen 5 und 15 Arme 120, 130 haben, die sich seitlich auf jeder Seite des Mittelteils 110 erstrecken.

Im Gebrauch kann jeder Arm 120, 130 einzeln und separat an einem Mikro-Hakenbefestigungselement 300 befestigt werden. In diesem Fall ist die Anzahl der Mikro-Hakenbefestigungselemente 300, die auf jeder Seite des Patienten P verwendet werden, gleich der Anzahl der Arme 120, 130 auf dieser Seite des Patienten P. Alternativ kann eine n:1 oder 1:n-Beziehung zwischen Armen 120, 130 und Mikro-Hakenbefestigungselemente 300 verwendet werden. Das heißt, ein Befestigungselement 300 kann zur Befestigung von zwei oder mehr Armen 120 bzw. 130 verwendet werden, oder ein Arm 120 bzw. 130 kann an zwei oder mehr Mikro-Hakenbefestigungselementen 300 befestigt werden.

Die Arme 120, 130 und die entsprechenden Mikro-Hakenbefestigungselemente 300 sind vorzugsweise so konfiguriert, dass sie einer Zugkraft von mindestens 40 N standhalten.

Um Zugang zu einem bestimmten Körperteil eines Patienten P zu ermöglichen, können ein oder mehrere Arme 120 bzw. 130 ungesichert bleiben oder sogar vom zentralen Abschnitt 110 abgetrennt werden, um während der Durchführung eines medizinischen Eingriffs einen zugänglichen Bereich zu schaffen. Der Vliesstoff des flächigen Elements 105 der Patienten-Immobilisationsvorrichtung 100 kann z.B. mit einer Sicherheitsschneidvorrichtung geschnitten werden, die eine in einem engen Schlitz angeordnete Klinge hat, in den ein Arm 120, 130 der Patienten-Immobilisationsvorrichtung 100 eingeführt werden kann, der aber für einen menschlichen Finger oder einen anderen Körperteil unzugänglich ist.

Perforationen 112, 113 können innerhalb des Mittelteils 110 gebildet werden, um die Mittelteile 110 der Patienten-Immobilisierungsvorrichtung 100 leicht abtrennbar zu gestalten. Wie gezeigt, können die Perforationen 112 senkrecht zur Längsachse des Mittelteils 110 zwischen zwei Armen 120, 130 verlaufen. Die Perforationen 112 erstrecken sich vorzugsweise parallel zur Haltekraft der Patienten-Immobilisierungsvorrichtung entlang der Arme 120, 130. Dadurch schwächen die Perforationen 112,113 nicht die Fähigkeit der Vorrichtung, einen Patienten P sicher zu halten. Die Perforationen 112, 113 bieten jedoch einen zusätzlichen Sicherheitsmechanismus für den Fall, dass ein Arm 120, 130 versehentlich oder mit einer zu hohen Zugkraft fixiert wurde, z.B. durch versehentliches Einklemmen. In diesem Fall kann der lose Arm 120 bzw. 130 in jede Richtung gezogen werden, ohne dass die Gefahr besteht, dass die übrigen Arme 120, 130 versehentlich entfernt werden. Der auch als Mittelteil 110 bezeichnete zentrale Abschnitt 110 reißt entlang der Perforationen 112, 113, so dass keine auf einen einzelnen Arm 120 bzw. 130 ausgeübte Kraft, unabhängig von deren Richtung, das Lösen eines benachbarten Arms 120 bzw. 130 verursachen kann.

Zur Förderung dieses Sicherheitsaspekts können die Perforationen 113 im Mittelteil 110 der Patienten-Immobilisierungsvorrichtung 100 tangential von den Armen 120, 130 in einem X-förmigen Muster über das Mittelteil 110 verlaufen.

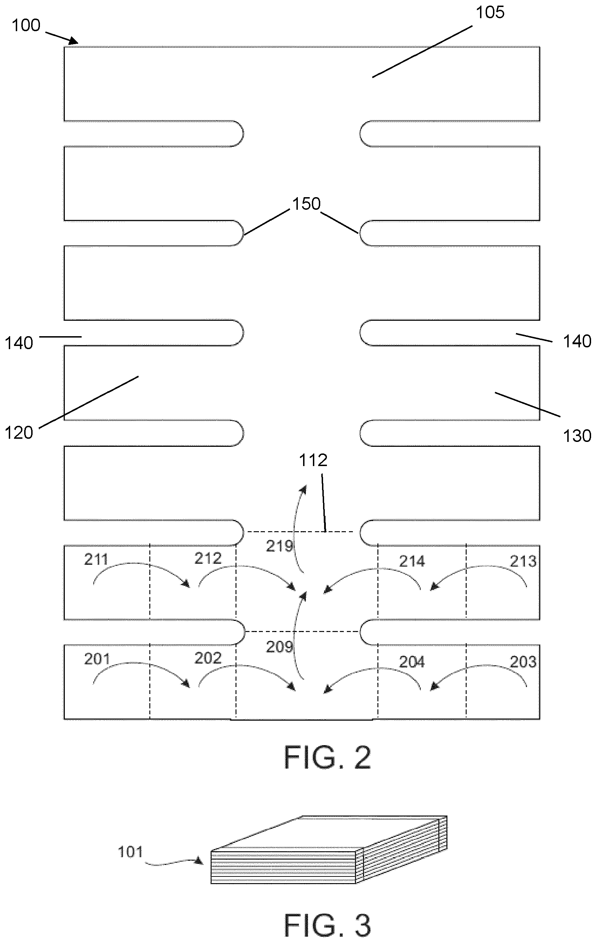

Die Patienten-Immobilisationsvorrichtung 100 wird vorzugsweise in gefaltetem Zustand 101 - wie in Figur 3 dargestellt - versandt und zur Verwendung durch medizinisches Fachpersonal am Einsatzort bereitgestellt. Wie in Figur 2 dargestellt, kann der gefaltete Zustand 101 erreicht werden, indem jeder Arm 120, 130 ein oder mehrere Male auf sich selbst gefaltet wird, wie durch die Pfeile 201, 202 und 203, 204 angegeben. Der derart in sich vorgefaltete Arm 120, 130 wird dann auf den mittleren Abschnitt 110 gefaltet. Wie durch Pfeile 209, 219 angezeigt, können die gefalteten Arme 120, 130 und ein entsprechend darunter liegender Teil des Mittelteils 110 entsprechend dem Pfeil 209 dann auf die nächsten entsprechend der Pfeile 211, 212 bzw. 213, 214 vorgefalteten Arme 120, 130 und den angrenzenden Teil des Mittelteils 110 gefaltet werden. Die Längsfaltung kann dabei bevorzugt mit den Perforationen 112 zusammenfallen. Der Faltvorgang ermöglicht es dem medizinischen Fachpersonal, die gefaltete Patienten-Immobilisationsvorrichtung 101 einfach zentral auf den Rumpf des Patienten P zu legen und dann dort zu entfalten, wie in Fig. 4 dargestellt. Während des Aufklappens kann das medizinische Fachpersonal die Arme 120, 130 an entsprechenden Mikro-Hakenbefestigungselementen 300 befestigen, die auf beiden Seiten des Patienten P an einem Bett oder Patiententisch 305 in einfacher Weise befestigbar sind. Auf diese Weise wird der Patient schnell zunehmend ruhig gestellt, ohne dass dabei irgendwelche komplizierten Schnitte am Gewebe vorgenommen werden müssten.

Ein Beispiel für ein Mikrohaken-Befestigungselement 300 ist in den Figuren 5 und 6 dargestellt. Das Mikrohaken-Befestigungselement 300 hat einen Körper mit einem im Allgemeinen flachen plattenförmigen vorderen Körperteil 310, das auch als Plattenteil 310 bezeichnet wird. Ein Mikrohaken-Pflaster 312 mit daran angeordneten Mikro-Haken 314 ist am vorderen Körperteil 310 befestigt. Das Mikrohaken-Befestigungselement 300 ist so konfiguriert, dass es an einer Schiene 301 eines Patiententisches 305 oder ähnlichem leicht einrastet. Im Zusammenhang mit dieser Spezifikation und den vorstehenden Ansprüchen wird jede Struktur, die einen Patienten stützt, als "Patiententisch" 305 bezeichnet. Dazu gehören unter anderem Operationstische, Betten, MRI/CT-Lagerflächen und dergleichen.

Das Mikro-Hakenbefestigungselement 300 umfasst einen oberen seitlichen Steg 332, der sich ungefähr senkrecht von der Rückseite des vorderen Körperteils 310 aus erstreckt. Ein Kanal 350 ist in einem unteren Steg 340 vorgesehen, der sich in einem Abstand von und parallel unter dem oberen seitlichen Steg 332 erstreckt. Der Kanal 350 ist etwa 1 cm breit, um einen unteren Teil der Schiene 301 aufzunehmen. Der untere Steg 340 erstreckt sich entlang eines unteren Endes des vorderen Körperteils 310 und kann eine allgemein V-förmige Querschnittsform haben. Der obere seitliche Steg 332 kann in einem Abstand von etwa 3 cm von der Bodenfläche des Kanals 350 angeordnet werden. Der untere Steg 340 und der obere Steg 332 sind so konfiguriert, dass sie häufig verwendete Schienen 301 aufnehmen können, die eine allgemein gebräuchliche rechteckige Querschnittsform und Abmessungen von beispielsweise 25 mm x 10 mm, 28,5 mm x 9,5 mm oder 31 mm x 7 mm haben können.

Das Mikro Haken-Pflaster 312 mit Mikro-Haken 314, das am vorderen Körperteil 310 befestigt ist, kann sich um ein unteres V-förmiges Ende des Mikro-Hakenbefestigungselement 300 legen und einen Teil des unteren Stegs 340 bedecken. Um die gewünschte Festigkeit zu erreichen, hat sich die Verwendung eines Mikrohaken-Pflasters 312 mit etwa zwischen 250 und 350 Mikrohaken pro cm², insbesondere mit etwa 300 Mikrohaken pro cm², als vorteilhaft erwiesen. Die Mikro-Haken 314 können besonders bevorzugt aus Polyamid oder Polypropylen hergestellt werden. Die Mikro-Haken 314 haben bevorzugt eine Höhe von etwa 0,5 mm, z.B. 0,4 mm. Das Mikro Haken-Pflaster 312 kann mit einer vorteilhaft aus Polyurethan gebildeten Klebeschicht 313 auf das Mikro-Hakenbefestigungselement 300 geklebt werden.

Ein elastisch verformbarer Gummistreifen, insbesondere ein Silikonkautschukstreifen 355, kann innerhalb des Kanals 350 angeordnet werden. Wenn das Mikro-Hakenbefestigungselement 300 an der Schiene 301 befestigt wird, wird ein unterer Teil der Schiene 301 innerhalb des Kanals 350 aufgenommen, während ein oberer Teil der Schiene 301 mittels des Silikonkautschukstreifens 355 gegen den oberen seitlichen Steg 332 gedrückt wird. Der Silikonkautschukstreifen 355 wird dabei elastisch verformt und sorgt für die erforderliche Klemmkraft für das sichere Halten des Mikro-Hakenbefestigungselements 300 an der Schiene 301.

Der Kanal 350 wird entlang einer Oberseite des unteren Stegs 340 zwischen dem Plattenteil 310 und einer parallelen Wand 339 gebildet. Eine Nut 338 kann innerhalb eines Bodens des Kanals 350 gebildet werden. Der verformbare Silikonkautschukstreifen 355 kann einen allgemein D-förmigen hohlen Querschnitt haben, wobei ein flacher Teil des D-förmigen Querschnitts auf einem Boden des Kanals 350 aufliegt und ein konvexer Teil des D-förmigen Querschnitts nach oben hin zur Schiene 301 zeigt. Ein Ankerteil 357 kann sich mittig von dem flachen Teil des D-förmigen Querschnitts vertikal in die Nut 338 erstrecken. Der verformbare Silikonkautschukstreifen 355 ähnelt einem D-förmigen Türdichtungsstreifen.

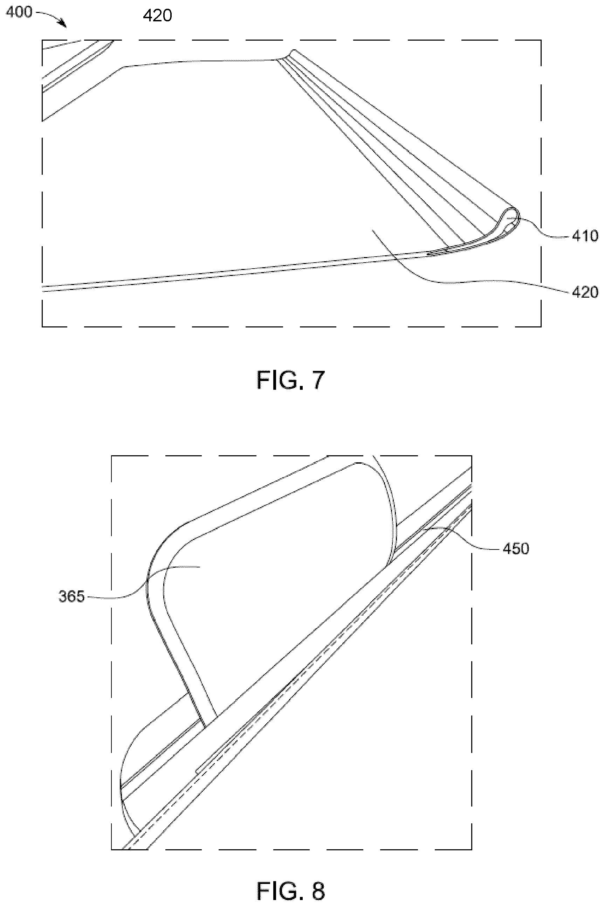

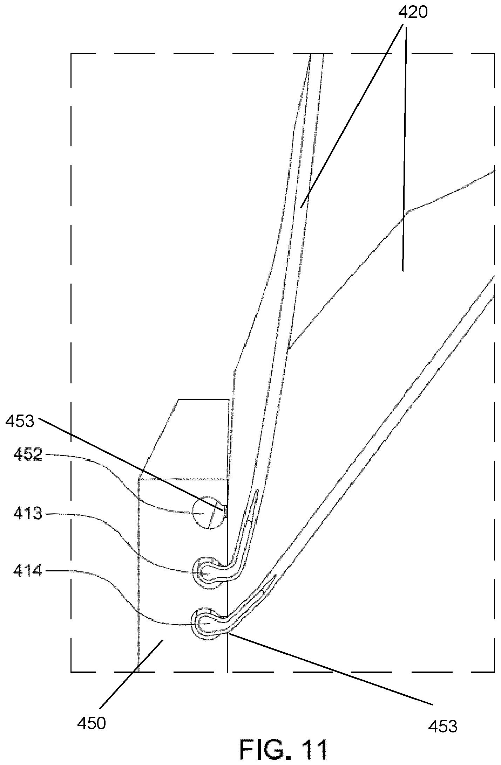

Unter Bezugnahme auf Figuren 7 bis11 werden nun alternative Ausführungsformen zu den Mikro-Hakenbefestigungselementen 300 gezeigt. Das abgebildete Alternative Befestigungselement 400 enthält ein Mikro-Hakenpflaster 420, das um eine Kederschnur 410 gewickelt ist. Das Befestigungselement 400 kann in eine Kederschiene 450 eines Patiententisches 305 eingesetzt werden. Die Kederschiene 450 verläuft in Längsrichtung an den Seiten des Patiententisches 305. Die Kederschiene 450 enthält wenigstens einen nutförmigen Hohlraum 452 (siehe Fig. 11), der durch einen nach oben oder zur Seite gerichteten Schlitz 453 zugänglich ist. Der Schlitz 453 ist breit genug, um das dünne Mikro-Hakenpflaster 420 in doppelter Lage aufzunehmen, und schmal genug, um dessen um die Kederschnur 410 gewickeltes Ende im nutförmigen Hohlraum 452 zu halten.

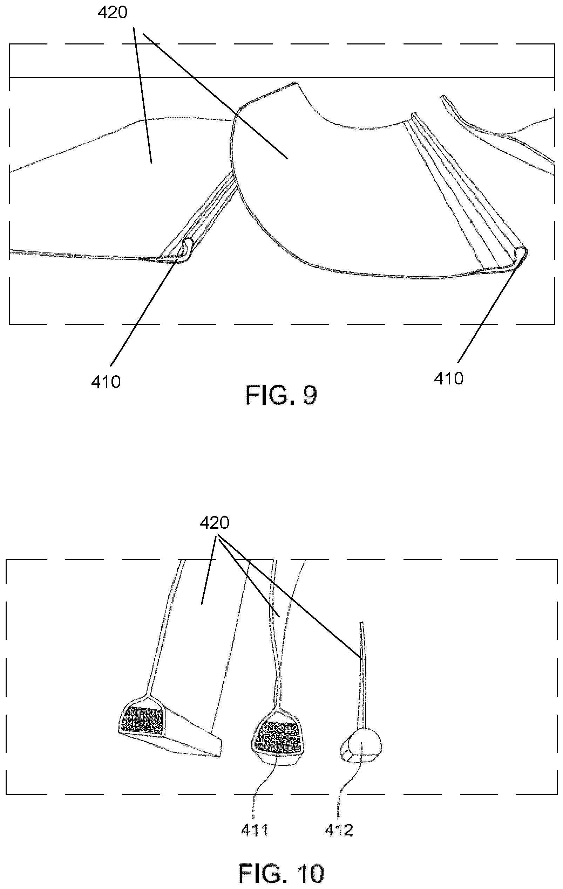

Wie in Figur 10 dargestellt, kann die Kederschnur 410 verschiedene Querschnittsformen haben. So kann sie beispielsweise einen polygonalen, runden, ovalen oder D-förmigen Querschnitt haben. Ein trapezförmiger Querschnitt 411 und ein allgemein D-förmiger Querschnitt 412 sind in Figur 10 dargestellt.

Wie in Figur 11 dargestellt, kann die Kederschnur 410 einen allgemein kreisförmigen unteren Teil, der im Hohlraum 452 der Kederschiene 450 gehalten wird, und einen dünneren oberen Teil umfassen, der durch den Schlitz 453 der Kederschiene 450 reicht. Der obere Teil der Kederschnur 410 kann gebogen werden, um das Mikro-Hakenpflaster 420 in einem definierten Winkel relativ zur Kederschiene 450 auszurichten. Wie in Fig. elf gezeigt, kann eine um etwa 90° gebogene Kederschnur 413 oder eine um etwa 45° gebogene Kederschnur 414 verwendet werden. Natürlich sind auch andere Winkel möglich.

Figur 8 zeigt das Befestigungselement 400 mit einem auf der Innenseite angeordneten Einwegpflaster 365. Ein solches Einwegpflaster 365 kann für verschiedene Zwecke und in Kombination mit jeder Art von Befestigungselement 300 bzw. 400 verwendet werden. Zum Beispiel kann das Einwegpflaster 365 verwendet werden, um die Mikrohaken 314 des Befestigungselements 300 oder 400 vor Schmutz zu schützen, wenn es nicht verwendet wird. In diesem Fall kann das Befestigungselement 300, 400 mit einem Einwegpflaster 365 versehen geliefert werden. Das Einwegpflaster 365 schützt die Mikrohaken 314 während des Transports und wird erst kurz vor der Verwendung entfernt.

Alternativ kann, wie in Figur 8 gezeigt, das Einwegpflaster 365 auch auf der patientenseitigen Seite des Befestigungselements 400 verwendet werden, um den direkten Kontakt zwischen den Mikrohaken und einem Patienten zu verhindern.

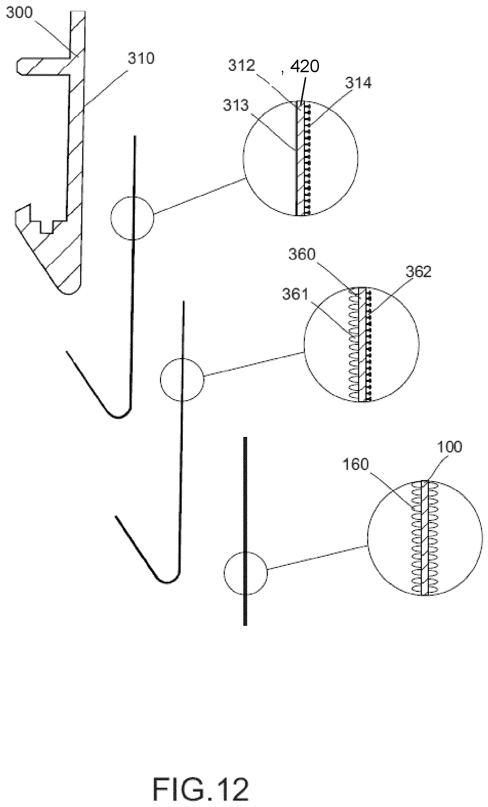

Figur 12 zeigt eine Querschnittsansicht eines Befestigungselements 300 in einer Explosionsdarstellung. Ein Mikro-Hakenpflaster 312 mit Mikrohaken 314 ist am vorderen Körperteil 310 des Mikrohaken-Befestigungselements 300 befestigt. Hier ist das Mikro-Hakenpflaster 312 durch eine Klebeschicht 313 mit dem vorderen Körperteil 310 des Mikrohaken-Befestigungselements 300 verklebt. Die Mikrohaken 314 sind auf einer gegenüberliegenden, nach außen gerichteten Seite des Mikro-Hakenpflasters 312 ausgebildet. Bei den Mikrohaken 314 kann es sich im Allgemeinen um pilzförmige Elemente handeln, die geeignet sind, in Schlaufen 160 des Vliesstoffs der Patienten-Immobilisationsvorrichtung 100 einzugreifen und sich in diesen fest zu klammern.

Das Mikro-Hakenpflaster 312 kann dauerhaft mit dem vorderen Körperteil 310 des Mikrohaken-Befestigungselements 300 verklebt werden und kann dann auch als Ankerpflaster 312 bezeichnet werden. Das Mikro-Hakenpflaster 312 ist in diesem Falle nicht leicht austauschbar.

Bei einigen Anwendungen ist es vorzuziehen, Einwegpflaster zu verwenden, die nicht gereinigt werden müssen. Ein solches Einwegpflaster 360 ist in Figur 12 dargestellt. Das Einwegpflaster 360 hat auf seiner Innenseite eine Schicht von Mikrofaserschlingen 361, die denen der Patienten-Immobilisierungsvorrichtung 100, 101 ähnlich sind. Auf seiner Außenseite hat das Einwegpflaster 360 eine Mikrohakenschicht 362, wobei die Mikrohaken denen des Ankerpflasters 312 entsprechen.

Das Einwegpflaster 360 kann so eine Zwischenschicht zwischen dem Ankerpflaster 312 und der Patienten-Immobilisiationsvorrichtung 100 bilden. Mehr als ein Einwegpflaster 360 kann zur Bildung einer Sandwich-Struktur verwendet werden, in der mehrere Einwegpflaster 360 zwischen dem Ankerpflaster 312 des Mikrohaken-Befestigungselements 300 und der Patienten-Immobilisationsvorrichtung 100 angeordnet sind. Nach jedem Gebrauch können die Patienten-Immobilisationsvorrichtung 100 und das äußerste Einwegpflaster 360 entsorgt werden, wodurch eine saubere und möglicherweise auch unmittelbar eine sterile Umgebung für den nächsten Patienten geschaffen wird.

Durch die Erfindung wird eine im klinischen oder ärztlichen Bereich sehr vorteilhafte, einfache und unkomplizierte Handhabung einer Patienten-Immobilisationsvorrichtung 100 bzw. 101 ermöglicht. Das flächige Element 105 mit seiner Vielzahl von Armen 120 bzw. 130 kann aufgrund seiner Materialbeschaffenheit unmittelbar mit den Mikro-Haken 314 am Mikro-Haken Befestigungselement 300, am Mikro-Haken Pflaster bzw. Ankerpflaster 312 oder 420 in Eingriff gebracht und fixiert werden. Da die die Mikro-Haken 314 aufweisenden, vorstehend genannten Befestigungselemente an der Schiene 301 oder am nutförmigen Hohlraum 452 eines Patiententisches 305 beliebig verschiebbar sind, lassen sich die Bedingungen für eine Ruhigstellung eines Patienten oder einzelner Körperteile des Patienten in extrem kurzer Zeit variabel herstellen.

Obwohl die vorliegende Erfindung mit Bezug auf beispielhafte Ausführungsformen beschrieben wurde, wird es für den Fachmann leicht ersichtlich sein, dass die Erfindung nicht auf die offengelegten oder illustrierten Ausführungsformen beschränkt ist, sondern im Gegenteil zahlreiche andere Modifikationen, Substitutionen, Variationen und weitreichende gleichwertige Regelungen abdecken soll, die im Umfang der folgenden Ansprüche enthalten sind. Insbesondere sind auch alle gezeigten und beschriebenen Details der verschiedenen Ausführungsformen beliebig untereinander kombinierbar oder austauschbar soweit sie im Umfang der Ansprüche enthalten sind.

### BEZUGSZEICHENLISTE

- 100: Patienten-Immoblisiationsvorrichtung
- 101: Patienten-Immobilisationsvorrichtung (in gefaltetem Zustand)
- 105: (flächiges) Element
- 110: längliches Mittelteil
- 112: Perforation
- 113: Perforation
- 120: linker Arm
- 130: rechter Arm
- 140: Längslücke (zwischen 120 oder 130)
- 150: Übergang (an 140)
- 160: Schlaufen (an 100, 101)

- 201: Pfeil
- 202: Pfeil
- 203: Pfeil
- 204: Pfeil
- 209: Teil (von 110)
- 211: Teil (von 120)
- 212: Teil (von 120)
- 213: Teil (von 130)
- 214: Teil (von 130)
- 219: Teil (von 110)

- 300: Mikro-Hakenbefestigungselement
- 301: Schiene
- 305: Patiententisch
- 310: vorderes Körperteil / Plattenteil
- 312: Mikro-Hakenpflaster/ Ankerpflaster
- 313: Kleberschicht
- 314: Mikro-Haken
- 332: oberer seitlicher Steg
- 338: Nut
- 339: Wand
- 340: unterer Steg
- 350: Kanal
- 355: Gummistreifen/ Silikonkautschukstreifen
- 357: Ankerteil
- 360: Einwegpflaster
- 361: Mikrofaser-Schlaufen
- 362: Mikrohaken-Schicht
- 365: Einweg-Pflaster

- 400: Befestigungselement
- 410: Kederschnur
- 411: Querschnitt (trapezförmig)
- 412: Querschnitt (D-förmig)
- 413: Kederschnur (90° gebogen)
- 414: Kederschnur (45° gebogen)
- 420: Mikro-Hakenpflaster
- 450: Kederschiene
- 452: Hohlraum/ Kedernut
- 453: Schlitz

- P: Patient

## Patentansprüche

1. Patienten-Immobilisationsvorrichtung (100, 101), aufweisend ein flächiges Element (105) aus einem nicht-gewebten Vliesstoff, wobei das flächige Element (105) einen länglichen Mittelteil (110) und eine Vielzahl von Armen (120, 130) hat, die sich seitlich auf beiden Seiten des Mittelteils (110) erstrecken.

2. Patienten-Immobilisationsvorrichtung (100) nach Anspruch 1 in einer gefalteten Aufbewahrungsposition (101), wobei jeder der Arme (120, 130) seitlich auf den länglichen Mittelteil (110) gefaltet ist und wobei Teile des langgestreckten Mittelteils (110) mit den darauf seitlich gefalteten Armen (120, 130) ihrerseits in Längsrichtung aufeinander gefaltet sind.

3. Patienten-Immobilisationsvorrichtung (100, 101) nach Anspruch 2, bei der jeder Arm (120, 130) zusätzlich seitlich auf sich selbst gefaltet und seitlich auf den länglichen Mittelteil (110) gefaltet ist.

4. System, umfassend eine Patienten-Immobilisationsvorrichtung (100, 101) nach einem der Ansprüche 1 bis 3 und eine Vielzahl von Mikro-Haken-Befestigungselemente (300) oder Befestigungselemente (400), die an wenigstens einer Seite eines Patiententisches (305) befestigbar sind.

5. System nach Anspruch 4, bei dem einer oder mehrere Arme (120, 130) der Patienten-Immobilisationsvorrichtung (100, 101) durch Mikro-Haken (314) gehalten werden, die an Teilen (310; 312; 420) der Mikro-Haken-Befestigungselemente (300) oder Befestigungselemente (400) angeordnet sind.

6. System nach Anspruch 4, bei dem jedes der Mikrohaken-Befestigungselemente (300) folgendes umfasst:
einen Körperteil (310) mit einer Vielzahl von Mikro-Haken (314), die darauf angeordnet sind;
einen oberen Steg (332); und
einen unteren Steg (340),
wobei der obere Steg (332) und der untere Steg (340) so konfiguriert sind, dass eine Schiene (301) eines Patiententisches (305) zwischen diesen einklemmbar ist.

7. System nach Anspruch 6, bei dem der untere Steg (340) einen Kanal (350) aufweist, der so konfiguriert ist, dass er einen unteren Teil der Schiene (301) des Patiententisches (305) lagert und das ferner einen elastisch verformbaren Gummistreifen (355) aufweist, der innerhalb des Kanals (350) anordenbar ist.

8. System nach einem der Ansprüche 4 bis 7, weiterhin umfassend Einwegpflaster (365), die zwischen der Patienten-Immobilisationsvorrichtung (100) und den Mikro-Hakenbefestigungselementen (300) angeordnet sind, wobei die Einwegpflaster (365) Stoffschlaufen aufweisen, die auf der Innenseite der Einwegpflaster (365) angeordnet sind und Mikrohaken (314), die an der Außenseite der Einwegpflaster (365) angeordnet sind.

9. System nach einem der Ansprüche 4 bis 8, bei dem jedes der Mikrohaken-Befestigungselemente (300) ein abnehmbares Einwegpflaster (360) aufweist, das in die Mikrohaken (314) der Mikrohaken-Befestigungselemente (300) eingreift.

10. System nach einem der Ansprüche 4 bis 9, bei dem jedes der Mikro-Haken-Befestigungselemente (300) oder Befestigungselemente (400) eine Kederschnur (410; 413; 414) und ein um die Kederschnur (410; 413; 414) gewickeltes Mikro-Hakenpflaster (420) aufweist.

11. System nach Anspruch 10, bei dem die Kederschnur (410; 413; 414) einen unteren Teil mit einem Querschnittsdurchmesser, der größer ist als der Durchmesser einer einen Hohlraum (452) bildenden Kedernut (452) eines Patiententisches (305) und einen oberen Teil mit einer Breite aufweist, die kleiner ist als die Weite eines Schlitzes (453) der Kedernut (452).

12. System nach Anspruch 11, bei dem ein oberer, durch den Schlitz (453) hindurchtretender Teil der Kederschnur (410; 413; 414) zwischen 45 Grad und 90 Grad gebogen ist.

13. Verfahren zur Sicherung eines Patienten auf einem Patiententisch, umfassend folgende Verfahrensschritte:
Bereitstellung der Patienten-Immobilisationsvorrichtung (100, 101) nach Anspruch 1;
Platzieren der Patienten-Immobilisationsvorrichtung (100; 110) auf dem Patienten P; und
Befestigung von zwei oder mehr Armen (120, 130) der Patienten-Immobilisationsvorrichtung (100) an Mikro-Hakenbefestigungselementen (300) oder Befestigungselementen (400) an gegenüberliegenden Seiten eines Patiententisches (305).

14. Verfahren nach Anspruch 13, das ferner umfasst:
Selektives Entfernen eines Teils der Patienten-Immobilisationsvorrichtung (100), um eine Zugangsöffnung zur Durchführung eines medizinischen Eingriffs am Patienten zu schaffen.

## Claims

1. Patient immobilization apparatus (100, 101) comprising a planar element (105) of a nonwoven fabric, the planar element (105) comprising an elongated middle part (110) and a plurality of arms (120, 130) extending laterally on both sides of the middle part (110).

2. The patient immobilization apparatus (100) according to claim 1 in a folded storage position (101) wherein each of the arms (120, 130) is folded laterally onto the elongated middle part (110) and wherein parts of the elongated middle part (110) with the arms (120, 130) folded laterally thereon are in turn folded longitudinally onto each other.

3. The patient immobilization apparatus (100, 101) according to claim 2, wherein each arm (120, 130) is additionally folded laterally upon itself and folded laterally upon the elongated middle part (110).

4. System comprising a patient immobilization apparatus (100, 101) according to any of claims 1 to 3 and a plurality of micro-hook fastening elements (300) or fastening elements (400) attachable to at least one side of a patient table (305).

5. The system of claim 4, wherein one or more arms (120, 130) of the patient immobilization apparatus (100, 101) are supported by micro-hooks (314) disposed on parts (310; 312; 420) of the micro-hook fastening elements (300) or fastening elements (400).

6. The system of claim 4, wherein each of the micro-hook-fastening elements (300) comprises:
a body part (310) comprising a plurality of micro-hooks (314) arranged thereon;
an upper web (332); and
a lower web (340),
wherein the upper web (332) and the lower web (340) are configured to clamp a rail (301) of a patient table (305) therebetween.

7. The system of claim 6, wherein the lower web (340) comprises a channel (350) configured to support a lower part of the rail (301) of the patient table (305) and further comprises a elastically deformable rubber strip (355) disposable within the channel (350).

8. The system of any of claims 4 to 7, further comprising disposable patches (365) arranged between the patient immobilization apparatus (100) and the micro-hook fastening elements (300), wherein the disposable patches (365) comprise fabric loops which are arranged on the inner face of the disposable patches (365) and micro-hooks (314) which are arranged on the outer face of the disposable patches (365).

9. The system of any of claims 4 to 8, wherein each of the micro-hook fastening elements (300) comprises a removable disposable patch (360) that engages the micro-hooks (314) of the micro-hook fastening elements (300).

10. The system of any of claims 4 to 9, wherein each of the micro-hook fastening elements (300) or fastening elements (400) comprises a keder cord (410; 413; 414) and a micro-hook patch (420) wrapped around the keder cord (410; 413; 414).

11. The system of claim 10, wherein the keder cord (410; 413; 414) comprises a lower part comprising a cross-sectional diameter greater than a diameter of a keder groove (452) forming a cavity (452) of a patient table (305) and an upper part comprising a width smaller than a width of a slot (453) of the keder groove (452).

12. The system of claim 11, wherein an upper part of the keder cord (410; 413; 414) passing through the slot (453) is bent between 45 degrees and 90 degrees.

13. Method of securing a patient on a patient table, comprising the following method steps:
providing the patient immobilization apparatus (100, 101) according to claim 1; placing the patient immobilization apparatus (100; 110) on the patient P; and attaching two or more arms (120; 130) of the patient immobilization apparatus (100) to micro-hook fastening elements (300) or fastening elements (400) on opposite sides of a patient table (305).

14. The method of claim 13, further comprising:
selectively removing a part of the patient immobilization apparatus (100) to provide an access opening for performing a medical procedure on the patient.

## Revendications

1. Dispositif d'immobilisation de patient (100, 101) comprenant un élément en forme de feuille (105) en tissu non tissé, l'élément en forme de feuille (105) ayant une partie centrale allongée (110) et une pluralité de bras (120, 130) s'étendant latéralement de chaque côté de la partie centrale (110).

2. Dispositif d'immobilisation de patient (100) selon la revendication 1 dans une position de stockage pliée (101), dans laquelle chacun des bras (120, 130) est plié latéralement sur la partie centrale allongée (110) et dans laquelle des parties de la partie centrale allongée (110) avec les bras (120, 130) pliés latéralement sur celle-ci sont à leur tour pliées longitudinalement les unes sur les autres.

3. Dispositif d'immobilisation de patient (100, 101) selon la revendication 2, dans lequel chaque bras (120, 130) est en outre replié latéralement sur lui-même et replié latéralement sur la partie centrale allongée (110).

4. Système comprenant un dispositif d'immobilisation de patient (100, 101) selon l'une des revendications 1 à 3 et une pluralité d'éléments de fixation à micro-crochets (300) ou d'éléments de fixation (400) configurés pour pouvoir être fixés sur au moins un côté d'une table de patient (305).

5. Système selon la revendication 4, dans lequel un ou plusieurs bras (120, 130) du dispositif d'immobilisation de patient (100, 101) sont maintenus par des micro-crochets (314) disposés sur des parties (310 ; 312 ; 420) des éléments de fixation de micro-crochets (300) ou des éléments de fixation (400).

6. Système selon la revendication 4, dans lequel chacun des éléments de fixation à micro-crochets (300) comprend :
une partie de corps (310) sur laquelle sont disposés une pluralité de micro-crochets (314);
une barre supérieure (332) ; et
une barre inférieure (340),
la barre supérieure (332) et la barre inférieure (340) étant configurées de manière à ce qu'un rail (301) d'une table de patient (305) puisse être serré entre elles.

7. Système selon la revendication 6, dans lequel la barre inférieure (340) comprend un canal (350) configuré pour supporter une partie inférieure du rail (301) de la table de patient (305) et qui comprend en outre une bande de caoutchouc élastiquement déformable (355) configurée pour pouvoir être placée à l'intérieur du canal (350).

8. Système selon l'une des revendications 4 à 7, comprenant en outre des pansements jetables (365) disposés entre le dispositif d'immobilisation de patient (100) et les éléments de fixation de micro-crochets (300), dans lequel les pansements jetables (365) comprennent des boucles de tissu disposées sur la face interne des pansements jetables (365) et des micro-crochets (314) disposés sur la face externe des pansements jetables (365).

9. Système selon l'une des revendications 4 à 8, dans lequel chacun des éléments de fixation à micro-crochets (300) comprend un pansement jetable amovible (360) qui s'engage dans les micro-crochets (314) des éléments de fixation à micro-crochets (300).

10. Système selon l'une des revendications 4 à 9, dans lequel chacun des éléments de fixation à micro-crochets (300) ou des éléments de fixation (400) comprend un cordon de passepoil (410 ; 413 ; 414) et un pansement à micro-crochets (420) enroulé autour du cordon de passepoil (410 ; 413 ; 414).

11. Système selon la revendication 10, dans lequel le cordon de passepoil (410 ; 413 ; 414) présente une partie inférieure dont le diamètre de la section transversale est supérieur au diamètre d'une rainure de passepoil (452) d'une table de patient (305) formant une cavité (452) et une partie supérieure dont la largeur est inférieure à la largeur d'une fente (453) de la rainure de passepoil (452).

12. Système selon la revendication 11, dans lequel une partie supérieure du cordon de passepoil (410; 413; 414) passant par la fente (453) est courbée entre 45 degrés et 90 degrés.

13. Procédé d'immobilisation d'un patient sur une table de patient, comprenant les étapes de procédé suivantes :
Mise à disposition du dispositif d'immobilisation d'un patient (100, 101) selon la revendication 1;
Mise en place du dispositif d'immobilisation d'un patient (100 ; 110) sur le patient P ; et
Fixation de deux ou plusieurs bras (120 ; 130) du dispositif d'immobilisation de patient (100) à des éléments de fixation à micro-crochets (300) ou à des éléments de fixation (400) sur des côtés opposés d'une table de patient (305).

14. Procédé selon la revendication 13, qui comprend en outre :
Retrait sélectif d'une partie du dispositif d'immobilisation d'un patient (100) afin de créer une ouverture d'accès pour effectuer une intervention médicale sur le patient.
